(19) Europäisches Patentamt
European Patent Office
Office européen des brevets

(11) Publication number: **0 278 542 B1**

(12) **EUROPEAN PATENT SPECIFICATION**

(45) Date of publication of patent specification: **06.04.94** (51) Int. Cl.$^5$: **C07D 257/04**

(21) Application number: **88200055.7**

(22) Date of filing: **13.01.88**

# (54) Preparation of tetrazole compounds.

(30) Priority: **14.01.87 GB 8700748**

(43) Date of publication of application:
**17.08.88 Bulletin 88/33**

(45) Publication of the grant of the patent:
**06.04.94 Bulletin 94/14**

(84) Designated Contracting States:
**AT BE CH DE ES FR GB GR IT LI LU NL SE**

(56) References cited:
**EP-A- 0 149 269**
**CA-A- 0 804 030**
**FR-A- 1 451 028**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Scott, Richard Mark**
**Hamlet Court**
**Hearts Delight**
**Borden New Sittingbourne Kent(GB)**
Inventor: **Fox, William**
**43 Glenfyan Avenue**
**Cilbarchan Renfrewshire, Pa10 2JV(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

This invention relates to the preparation of certain 1-phenyl-5-phenoxy-1H-tetrazole compounds which are useful as herbicides. Such compounds are disclosed as being herbicidally active in European Patent Application Publication No. 149269A.

The laboratory-scale preparation of the compounds, as described in EP-A-149269, involves the preparation of a 1-phenyl-5-chloro-1H-tetrazole intermediate, by reaction of a phenyl isocyanodichloride with sodium azide, followed by reaction with a phenol under dry conditions.

In Canadian Patent Specification No. 804030 there is described the preparation of 1-(p-chlorophenyl)-5-phenoxy-1H-tetrazole by reaction of p-chlorophenyl-isocyanodichloride with phenol, followed by reaction of the product with sodium azide. The first step is carried out under anhydrous conditions, careful pre-drying of the phenol being carried out.

The applicants have now discovered a process for preparing tetrazole compounds of the above mentioned European Patent Application Publication No. 149 269A which is more suitable for industrial practice than the prior processes referred to above.

According to the present invention there is provided a process for the preparation of a compound of the general formula I

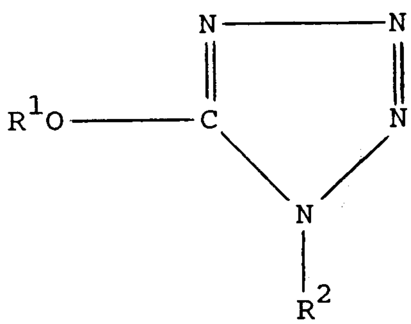

(I)

wherein each of $R^1$ and $R^2$ independently represents an optionally substituted phenyl group, in which process a compound of the general formula II

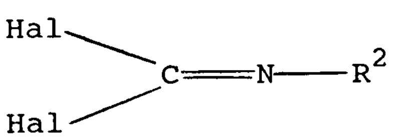

(II)

wherein each Hal represents a halogen atom, is reacted with a compound of the general formula $R^1OH$, or a phenoxide derivative thereof, in the presence of a solvent comprising water and, when the reaction mixture is two-phase, in the presence of a phase transfer catalyst; and the product is reacted with an inorganlc azide.

The solvent for the first step may be water alone or may comprise water and one or more organic components. The water content can be very low. For example, the phenoxide (when used as such) can be applied as a slurry, although it is preferred that sufficient water to dissolve the phenoxide is used. The organic component(s) optionally present may serve to promote the stirrability of the reaction mixture and may aid the dissolution of the products formed. The ratio of water to organic component(s) is preferably in the range 1:10 to 1:0 by volume. Preferably, the solvent is water alone or, most preferably a two-phase solvent comprising water and one organic component, suitably an alcohol, ketone, ether, or preferably, a hydrocarbon or chlorinated hydrocarbon. Toluene and, especially 1,2-dichloroethane, are especially suitable for large-scale manufacture. The reaction mixture commonly forms two phases, even when no organic component is present in the solvent, and for such a reaction mixture a phase transfer catalyst is employed. Preferred phase-transfer catalysts are quaternary ammonium salts, especially halides, for example benzyltriethylammonium chloride, Adogen 464 (trade mark for a trifatty monomethyl quaternary ammonium chloride) or, especially, tetrabutylammonium bromide. The catalyst is suitably present in an amount from 0.1 to 10 mol %, based on the compound of formula II, preferably 0.5 to 2 mol %.

The solvent/catalyst conditions described in the foregoing paragraph with regard to the first step are suitable for the second step, the azide reaction. Thus, whilst the product of the first step may be isolated before the azide step, if desired, such isolation is not necessary and the azide reaction is preferably carried out on the product of the first step without isolation thereof. When the reaction mixture for the first step is two-phase it may be convenient to separate the organic phase prior to the second step, for example to make best use of available plant capacity, but the second step proceeds efficiently without such separation, an aqueous solution of the azide simply being added to the complete reaction mixture produced in the first step.

Preferably, the phenol compound is added as an alkali metal, for example lithium, potassium or, especially, sodium, phenoxide, in water, whilst the compound of formula II is added in an organic solvent.

A suitable temperature range for each step is 0 °C to 100 °C, preferably 10 °C to 60 °C. Each step proceeds efficiently without application of heat. The reactions which have been carried out are exothermic. Cooling may be effected, if necessary, to keep the temperature within desired limits.

The inorganic azide may be a salt of hydrazoic acid, for example ammonium azide, an alkaline earth metal azide, or, especially, an alkali metal azide. Sodium azide is preferred.

In the definitions given above, and because of the high level of herbicidal activity associated with the product of formula I which contains such substituents, $R^1$ is preferably a phenyl group optionally substituted by a halogen atom or an amino group, or a $C_{1-6}$alkyl, alkoxy or haloalkyl, especially methyl, isopropyl, methoxy or trifluoromethyl, group; and $R^2$ is preferably a phenyl group optionally substituted by a halogen atom, a nitro group, or a $C_{1-6}$alkyl, haloalkyl or haloalkylsulphonyl, especially methyl, trifluoromethyl or trifluoromethylsulphonyl, group. It is preferred that the phenyl ring $R^1$ is monosubstituted or, especially, unsubstituted and that the phenyl ring $R^2$ is either unsubstituted or bears at least one substituent, preferably trifluoromethyl, at the 3-position. In one especially preferred compound $R^1$ represents phenyl and $R^2$ represents 3-trifluoromethylphenyl.

In the above definitions, halogen atoms in compounds of formula I, as direct substituents of a phenyl ring or in haloalkyl or haloalkylsulphonyl substituents thereof, are preferably chlorine or fluorine atoms. The halogen atoms denoted Hal in the definition of the compound of formula II are preferably chlorine atoms.

The following examples illustrate the invention. Analyses were carried out by gas chromatography (GC).

## Example 1

### Preparation of 1-(3′-trifluoromethylphenyl)-5-phenoxy-1H-tetrazole

A 50 litre glass reactor was equipped with a water-bath, stirrer, thermometer in a thermometer well, bottom outlet, addition funnel and a condenser.

The reactor was charged with 1,2-dichloroethane (17.5 litres), 3-trifluoromethylphenyl isocyanodichloride (12.36kg, 98% based purity, 50 mol), and tetra-n-butylammonium bromide (161.2g, 1 mol % based on the isocyanodichloride).

A solution of phenol (4.73kg, 50 mol) in aqueous sodium hydroxide (2.01kg, 50 mol, in 4.5 litres of deionised water) was added to the reactor over a period of 75 minutes with the cooling bath at 20 °C. The internal temperature rose from 23 to 42 °C during this addition. After stirring for a further 30 minutes a sample was removed and GC analysis indicated that 4.3% of the isocyanodichloride remained unconverted. Therefore, a further quantity of phenoxide (203g phenol and 86g of sodium hydroxide in 250ml deionised water) was added dropwise over 1 hour. This reduced the amount of unconverted isocyanodichloride to 0.5% and the amount of the desired intermediate isocyanochloride product to 93.6%.

Stirring was continued whilst a solution of sodium azide (3.2kg, 47.5 mol) in 0.01M sodium hydroxide solution (10.4 litres) was added dropwise over a period of 4 hours. The reaction was mildly exothermic rising to a maximum of 33 °C with the bath maintained at 15-20 °C. After a further hour the reaction was no longer exothermic but GC analysis indicated approximately 5% of unconverted intermediate. Therefore, a further quantity of sodium azide (230g) dissolved in 0.01M sodium hydroxide (770ml) was added and the mixture stirred for a further 90 minutes.

Stirring was discontinued and the heavy organic phase separated and transferred to a distillation apparatus fitted with a fractionating column. Some of the 1,2-dichloroethane was distilled off and isopropyl alcohol was added to form an azeotrope with the remaining 1,2-dichloroethane. Once all the 1,2-dichloroethane had been removed further isopropyl alcohol was added, bringing the total amount of alcohol added to approximately 22 litres. Water (10 litres) was added and the mixture cooled to crystallise out the product, which was filtered off and washed with 1:1 isopropyl alcohol/water mixture (2 x 1.5 litres) and finally hexane before drying.

| | |
|---|---|
| Yield : | 14.12kg; white crystals, mp = 73-74 °C |
| Purity : | 97% ±2% |
| Overall yield from the isocyanodichloride : | 89.4% |

Example 2

Preparation of 1-(3′-trifluoromethylphenyl)-5-phenoxy-1H-tetrazole

A 3-necked 100ml flask equipped with a thermometer, addition funnel and condenser was charged with demineralised water (20mls), tetrabutylammonium bromide (0.16g) and 3-trifluoromethylphenyl isocyanodichloride (12.28g at 98.5% purity). Phenol was dissolved in sodium hydroxide and added dropwise to the reaction mixture over 20 minutes at 25-30 °C. The mixture was stirred for a further 60 minutes. Following GC analysis further sodium phenoxide (5 mol %) was added and the reaction mixture stirred for a further 15 minutes. A further GC analysis indicated 98% conversion of the isocyanodichloride intermediate.

Sodium azide (12.35mls, 25% w/v) was added dropwise over 5 minutes and stirred for a further 70 minutes. Following GC analysis, further tetrabutylammonium bromide was added (0.64g in 10ml water) and the reaction mixture stirred vigorously for a further 30 minutes. GC analysis indicated 97% conversion. A white solid had formed and the reaction mixture was heated to 70 °C to form an oil, cooled, filtered and recrystallised from isopropyl alcohol/water.

Yield = 7.2g, mp = 71-72 °C.

Summary of Further Experiments

The following table summarises further experiments which were carried out. Yields were determined by GC analysis. All experiments were one-pot reactions in which 3-trifluoromethylphenyl isocyanodichloride (ICD) and phenol were employed as starting materials, in the presence of a base.

| BASE | SOLVENT | TIME mins | TEMP °C | MOLE RATIO phenol/ICD | MOLE RATIO base/ICD | CATALYST | YIELD OF TETRAZOLE |
|---|---|---|---|---|---|---|---|
| NaOH | EDC[1] | 30 | reflux | 1.65 | 1.55 | TBAB[2] | 93.0% |
| NaOH | EDC | 255 | 24-29 | 1.65 | 1.50 | TBAB | 95.2% |
| NaOH | EDC | 180 | 24-29 | 1.00 | 1.00 | TBAB | 95.2% |
| LiOH | EDC | 180[3] | 24-29 | 1.10 | 1.05 | TBAB | 93.1% |
| LiOH | toluene | 645 | 24-29 | 1.1 | 1.05 | TBAB | 88.5% |
| LiOH | EDC | 480 | 24-29 | 1.1 | 1.05 | BTEACL[4] | 94.7% |
| LiOH | EDC | 20 | 24-29 | 1.1 | 1.05 | AD464[5] | 84.2% |
| LiOH | toluene | 40 | 24-29 | 1.1 | 1.05 | AD464 | 93.0% |
| NaOH | EDC | 80 | 24-29 | 1.02 | 0.95 | TBAB | 95.0% |
| LiOH | EDC | 310 | 24-29 | 1.02 | 1.00 | TBAB | 95.5% |
| LiOH | EDC | 240 | 24-29 | 1.02 | 0.95 | AD464 | 94.8% |
| NaOH | toluene | 300 | 24-29 | 1.02 | 0.95 | TBAB | 93.8% |

**Notes**

1: 1,2-dichloroethane
2: tetrabutylammonium bromide
3: the TBAB was added after 150 minutes;
4: benzyltriethylammonium chloride;
5: Adogen 464 (trade mark)

## Claims

1. A process for the preparation of a compound of the general formula I

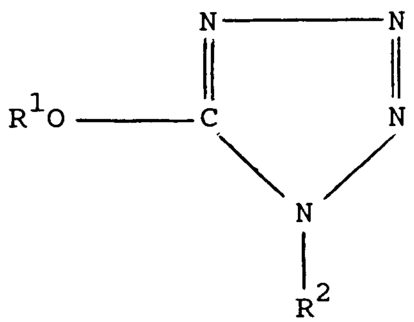

(I)

wherein each of $R^1$ and $R^2$ independently represents an optionally substituted phenyl group, in which process a compound of the general formula II

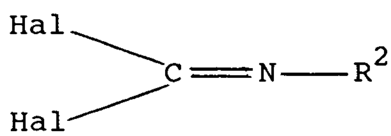

(II)

wherein each Hal represents a halogen atom, is reacted with a compound of the general formula $R^1OH$, or a phenoxide derivative thereof, in the presence of a solvent comprising water and, when the reaction mixture is two-phase, in the presence of a phase transfer catalyst; and the product is reacted with an inorganic azide.

2. A process according to claim 1 wherein said solvent is water alone or water and one or more organic components, the ratio of water to organic component(s) being in the range 1:10 to 1:0 by volume.

3. A process according to claim 2 wherein said solvent comprises water and one organic component.

4. A process according to claim 3 wherein said solvent is water/1,2-dichloroethane.

5. A process according to any one of the preceding claims wherein each step is carried out at a temperature in the range 10 °C to 60 °C.

6. A process according to any one of the preceding claims wherein the azide is an alkali metal azide.

7. A process according to any one of the preceding claims wherein the azide reaction is carried out on the product of the first step without isolation thereof.

8. A process according to any preceding claim wherein $R^1$ is a phenyl group optionally substituted by a halogen atom or an amino group, or a $C_{1-6}$alkyl, alkoxy or haloalkyl group; and $R^2$ is a phenyl group optionally substituted by a halogen atom, a nitro group, or a $C_{1-6}$alkyl, haloalkyl or haloalkylsulphonyl group.

9. A process according to claim 8 wherein $R^1$ represents a phenyl group and $R^2$ represents a 3-trifluoromethylphenyl group.

## Patentansprüche

1. Verfahren zur Herstellung einer Verbindung der allgemeinen Formel I

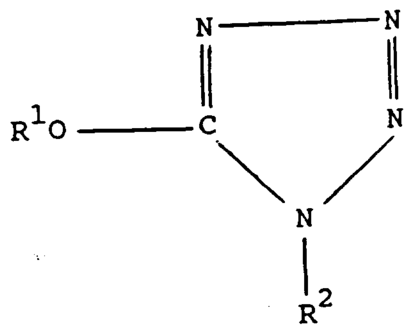

(I),

worin jeder Rest $R^1$ und $R^2$ unabhängig voneinander eine gegebenenfalls substituierten Phenylgruppe darstellt, in welchem Verfahren eine Verbindung der allgemeinen Formel II

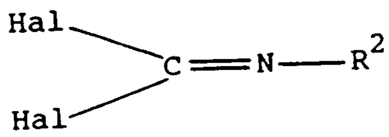

(II),

worin jeder Rest Hal ein Halogenatom bedeutet, mit einer Verbindung der allgemeinen Formel $R^1OH$ oder einem Phenoxidderivat hievon in Anwesenheit eines Wasser umfassenden Lösungsmittels und, wenn das Reaktionsgemisch zweiphasig ist, in Anwesenheit eines Phasentransferkatalysators umgesetzt wird; und das Produkt mit einem anorganischen Azid umgesetzt wird.

2. Verfahren nach Anspruch 1, worin das Lösungsmittel nur Wasser ist oder Wasser mit einer oder mehreren organischen Komponenten ist, wobei das Verhältnis von Wasser zur organischen Komponente bzw. zu den organischen Komponenten im Bereich von 1:10 bis 1:0, auf das Volumen bezogen, liegt.

3. Verfahren nach Anspruch 2, worin das Lösungsmittel Wasser und eine organische Komponente umfaßt.

4. Verfahren nach Anspruch 3, worin das Lösungsmittel Wasser/1,2-Dichlorethan ist.

5. Verfahren nach einem der vorstehenden Ansprüche, worin jede Stufe bei einer Temperatur im Bereich 10 °C bis 60 °C ausgeführt wird.

6. Verfahren nach einem der vorstehenden Ansprüche, worin das Azid ein Alkalimetallazid ist.

7. Verfahren nach einem der vorstehenden Ansprüche, worin die Azidreaktion an dem Produkt der ersten Stufe ohne Isolierung hievon ausgeführt wird.

8. Verfahren nach einem vorstehenden Anspruch, worin $R^1$ eine gegebenenfalls durch ein Halogenatom oder eine Aminogruppe oder eine $C_{1-6}$Alkyl-, Alkoxy- oder Halogenalkoxygruppe substituierte Phenylgruppe ist; und $R^2$ eine gegebenenfalls durch ein Halogenatom,eine Nitrogruppe oder eine $C_{1-6}$Alkyl-, Halogenalkyl- oder Halogenalkylsulfonylgruppe substituierte Phenylgruppe ist.

9. Verfahren nach Anspruch 8, worin $R^1$ eine Phenylgruppe darstellt und $R^2$ eine 3-Trifluormethylphenylgruppe bedeutet.

**Revendications**

1. Un procédé pour la préparation d'un composé de la formule générale I

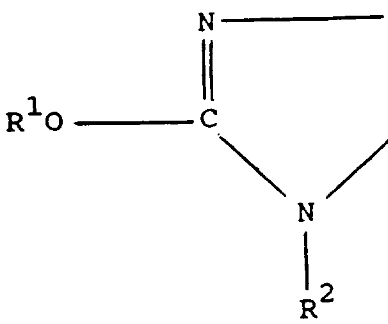

(I)

dans laquelle chacun des $R^1$ et $R^2$ représente indépendamment un groupe phényle facultativement substitué, procédé dans lequel un composé de la formule générale II

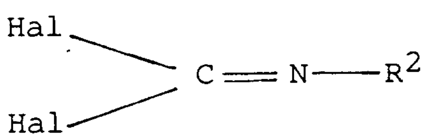

(II)

dans laquelle chaque Hal représente un atome d'halogène, est mis à réagir avec un composé de la formule $R^1OH$, ou un dérivé phénoxyde de celui-ci, en présence d'un solvant comportant de l'eau et, lorsque le mélange réactionnel est à deux phases, en présence d'un catalyseur de transfert de phase, après quoi on fait réagir le produit avec un azide inorganique.

2. Un procédé selon la revendication 1, dans lequel ledit solvant est uniquement de l'eau ou de l'eau et un ou plus d'un composant organique, le rapport de l'eau au(x) composant(s) organique(s) se situant dans la gamme de 1:10 à 1:0 en volume.

3. Un procédé selon la revendication 2, dans lequel ledit solvant comprend de l'eau et un composant organique.

4. Un procédé selon la revendication 3, dans lequel ledit solvant est un mélange eau/1,2-dichloroéthane.

5. Un procédé selon l'une quelconque des revendications précédentes, dans lequel chaque étape est mise en oeuvre à une température dans la gamme de 10 ° C à 60 ° C.

6. Un procédé selon l'une quelconque des revendications précédentes, dans lequel l'azide est un azide de métal alcalin.

7. Un procédé selon l'une quelconque des revendications précédentes, dans lequel la réaction à l'azide est réalisée sur le produit de la première étape sans isolement de celui-ci.

8. Un procédé selon l'une des revendications précédentes, dans lequel $R^1$ est un groupe phényle facultativement substitué par un atome d'halogène ou un groupe amino, ou un groupe alkyle en $C_1$ à $C_6$, alkoxy en $C_1$ à $C_6$, ou haloalkyle en $C_1$ à $C_6$, et $R^2$ et un groupe phényle facultativement substitué par un atome d'halogène, un groupe nitro ou un groupe alkyle en $C_1$ à $C_6$, haloalkyle en $C_1$ à $C_6$, ou haloalkylsulfonyle en $C_1$ à $C_6$.

9. Un procédé selon la revendication 8 dans lequel $R^1$ représente un groupe phényle, et $R^2$ représente un groupe 3-trifluorométhylphényle.